(19)

Europäisches
Patentamt

European
Patent Office

Office européen
des brevets

(11) **EP 4 336 511 A1**

(12) **EUROPEAN PATENT APPLICATION**

(43) Date of publication:
**13.03.2024 Bulletin 2024/11**

(21) Application number: **23194655.9**

(22) Date of filing: **31.08.2023**

(51) International Patent Classification (IPC):
**G16H 20/17** (2018.01)

(52) Cooperative Patent Classification (CPC):
**G16H 40/40; G16H 20/17; G16H 50/30;
G16H 50/50**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.09.2022 US 202263374311 P**

(71) Applicant: **Insulet Corporation
Acton MA 01720 (US)**

(72) Inventors:
• **LEE, Joon Bok
Acton (US)**
• **CAUSEY, James
Simi Valley (US)**
• **D'ARCO, John
Wilmington (US)**
• **O'CONNOR, Jason
Acton (US)**

(74) Representative: **Peterreins Schley
Patent- und Rechtsanwälte PartG mbB
Hermann-Sack-Straße 3
80331 München (DE)**

(54) **METHOD FOR DETECTING OCCLUSIONS IN A FLUID PATH USING BLOOD GLUCOSE
READINGS**

(57) Disclosed herein is a system and method implementing a software-based method for detecting or confirming the detection of occlusions in the fluid path of an automatic drug delivery system. The invention uses real time glucose readings from a continuous glucose monitor in collaboration with past insulin delivery history and user indications of ingestion of carbohydrates, to determine cases where the user's glucose concentration is not being sufficiently impacted by the expected insulin delivery, which may indicate a significant pump site issue.

FIG. 1

**Description**

**BACKGROUND**

Many conventional automated drug delivery (ADD) systems are well known, including, for example, wearable drug delivery devices. The drug delivery device can be designed to deliver any type of liquid drug to a user. In specific embodiments, the drug delivery device can be, for example, an OmniPod® drug delivery device manufactured by Insulet Corporation of Acton, Massachusetts. The drug delivery device can be a drug delivery device such as those described in U.S. Pat. No. 7,303,549, U.S. Pat. No. 7,137,964, or U.S. Pat. No. 6,740,059, each of which is incorporated herein by reference in its entirety.

Such drug delivery devices typically include a positive displacement pumping mechanism to force a liquid drug from a reservoir through a fluid path to the patient. The fluid path typically comprises a flexible tube or needle coupled, at one end, to the reservoir. The other end of the fluid path is coupled to a cannula which is inserted under the skin of the patient for delivery of the liquid drug. The cannula may be inserted via a needle mechanism wherein a needle/cannula combination is forced by an actuator into the skin of the user and thereafter the needle is withdrawn, leaving the cannula in place. The cannula may have one or more ports defined on a distal end thereof through which the liquid drug is dispensed.

It is not uncommon for the one or more ports to become fully or partially clogged or occluded. The occlusions may be caused, for example, by body fluids, immunoglobulins, or protein aggregates, or even insulin aggregates, formed as the insulin denatures, which accumulate in the fluid path. Most commonly, the occlusions occur at or near the distal end of the cannula and may clog the one or more ports. Less commonly, the occlusions occur at other points along the fluid path. The occlusions may reduce the amount of the liquid drug that can be dispensed or may potentially preclude dispensing any quantity of the liquid drug.

Occlusions occurring at insulin delivery sites (pump sites) is a major challenge in ensuring safe and effective insulin delivery for people with diabetes that utilize subcutaneous insulin pumps. For continuous subcutaneous insulin infusion, a reduction in the insulin delivery due to a fluid occlusion can cause harm in people managing diabetes. The venous glucose of a T1 diabetic, for example, can change at a rate of up to about 1 mg/dL/minute. In the absence of a steady basal delivery of insulin, the patient may quickly become hyperglycemic.

Thus, it would be desirable to be able to detect when occlusions that reduce or stop the delivery of insulin occur, so as to enable the drug delivery system to take remedial action, or, alternatively, warn the user of a potentially dangerous situation, such that the user can take remedial action.

**SUMMARY**

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one embodiment of the invention, disclosed herein is an automatic drug delivery (ADD) system which is designed to detect or confirm the detection of occlusions in the fluid path and a software-based method for detecting or confirming the detection of occlusions in the fluid path of an automated drug delivery system. The invention proposes the use of real time glucose readings from a continuous glucose monitor in collaboration with past insulin delivery history, and, where available, user indications of ingestion of carbohydrates, to determine cases where the user's glucose concentration is not being sufficiently impacted by the expected insulin delivery, which may indicate a significant pump site issue.

A hardware-based occlusion sensor was disclosed in U.S. Provisional Patent Application No. 63/359,010, filed July 7, 2022, the contents of which are incorporated herein by reference in their entirety. The software-based method disclosed herein can be used in lieu of hardware-based methods or can augment hardware-based methods to confirm indications of an occlusion.

**BRIEF DESCRIPTION OF THE DRAWINGS**

In the drawings, like reference characters generally refer to the same parts throughout the different views. In the following description, various embodiments of the present invention are described with reference to the following drawings, in which:

FIG. 1 illustrates a functional block diagram of an exemplary system suitable for use with the devices disclosed herein.
FIG. 2 is a block diagram of the described system and method.
FIG. 3 is a flowchart showing the software-based process for detecting occlusions in the fluid path.

**DETAILED DESCRIPTION**

Various embodiments of the present invention include systems and methods for delivering a medication to a user using a drug delivery device, either autonomously, and/or in accordance with a wireless signal received from an electronic device. In various embodiments, the electronic device may be a user device comprising a smartphone, a smart watch, a smart necklace, a module attached to the drug delivery device, or any other type or sort of electronic device that may be carried by the user or worn on the body of the user and that executes an algorithm that computes the times and dosages of delivery of the medication. Alternatively, the drug delivery device operates an algorithm stored on the drug delivery device itself, without reliance on a remote electronic device for delivering medicament to the user.

For example, the user device or drug delivery device may execute an "artificial-pancreas" (AP) algorithm that computes the times and dosages of delivery of insulin. The user device and/or drug delivery device may also be in communication with a sensor, such as a glucose sensor or a continuous glucose monitor (CGM), that collects data on a physical attribute or condition of the user, such as a glucose level. The sensor may be disposed in or on the body of the user and may be part of the drug delivery device or may be a separate device.

Alternatively, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the drug delivery device). In these embodiments, the drug delivery device contains computing hardware (e.g., a processor, memory, firmware, etc.) that executes some or all of the algorithm that computes the times and dosages of delivery of the medication.

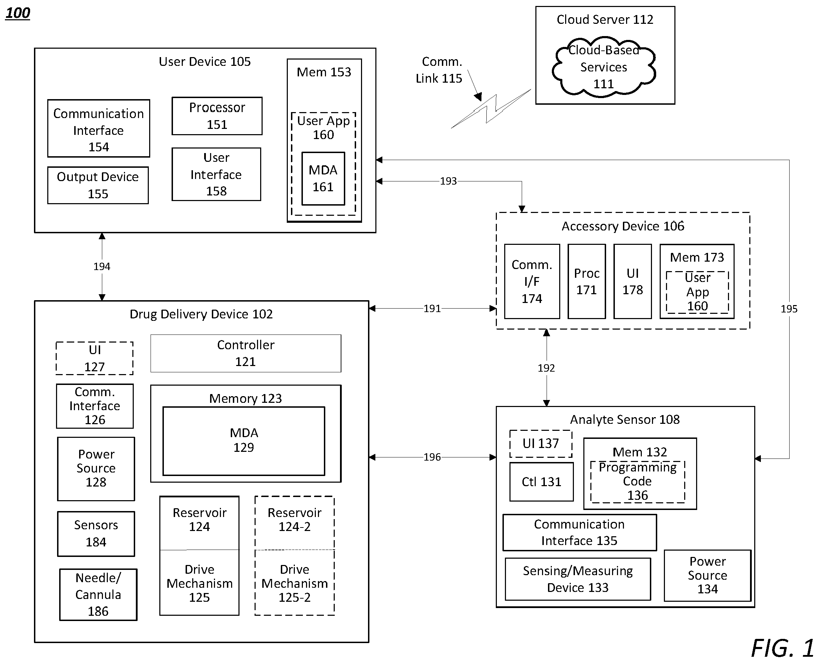

FIG. 1 illustrates a functional block diagram of an exemplary drug delivery system 100 suitable for implementing the systems and methods described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The drug delivery system 100 may be an automated drug delivery system that may include a drug delivery device 102 (which may be wearable), an analyte sensor 108 (which may also be wearable), and a user device 105. As will be discussed below, the drug delivery system may also comprise an accessory device 106 and/or a cloud server 112.

It should be understood that all computation steps, software steps, control steps and/or algorithm steps described in this disclosure may be carried out by a processor in a single device of the system, i.e. by a processor in the drug delivery device, by a processor in the user device, by a processor in the analyte sensor, by a processor in the accessory device or by a processor of the cloud server. Alternatively, the computation steps, software steps, control steps and algorithm steps described in this disclosure may be performed by two or more processors in the mentioned devices, i.e. in the drug delivery device, in the user device, in the analyte sensor, in the accessory device and/or in the cloud server. As these devices can communicate with each other, it is generally irrelevant which of these processors perform the respective steps.

Drug delivery system 100, in an optional example, may also include an accessory device 106, such as a smartwatch, a personal assistant device, a smart insulin pen, or the like, which may communicate with the other components of system 100 via either a wired or wireless communication links 191-193.

*User Device*

The user device 105 may be a computing device such as a smartphone, a smartwatch, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 incorporating medication delivery algorithm (MDA) 161 to manage a user's blood glucose levels and for controlling the delivery of the drug, medication, or therapeutic agent to the user, as well for providing other functions, such as calculating carbohydrate-compensation dosage, a correction bolus dosage and the like as discussed below. The user device 105 may be used to activate, deactivate, trigger a needle/canula insertion, program, adjust settings, and/or control operation of drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 106. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, and the like. The user interface 158 may be under the control of the processor 151 and be configured to present a graphical user interface that enables the input of a meal announcement, adjust setting selections and the like as described herein.

In a specific example, when the user app 160 includes MDA 161, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a real-time basal dosage according to a diabetes treatment plan. In addition, as a MDA 161, user app 160 provides functionality to output signals to the drug delivery device 102 via communications interface 154 to deliver the determined bolus and/or basal dosages.

The communication interface 154 may include one or more transceivers that operate according to one or more radio-frequency protocols. In one embodiment, the transceivers may comprise a cellular transceiver and a Bluetooth® transceiver. The communication interface 154 may be configured to receive and transmit signals containing information usable by user app 160.

User device 105 may be further provided with one or more output devices 155 which may be, for example, a speaker or a vibration transducer, to provide various signals to the user.

*Drug Delivery Device*

In various exemplary embodiments, drug delivery device 102 may include a reservoir 124 and drive mechanism 125, which are controllable by controller 121, executing a medication delivery algorithm (MDA) 129 stored in memory 123, which may perform some or all of the functions of the AP application described above, such that user device 105 may be unnecessary for drug delivery device 102 to carry out drug delivery and control. Alternatively, controller 121 may act to control reservoir 124 and drive mechanism 125 based on signals received from user app 160 executing on a user device 105 and communicated to drug delivery device 102 via communication link 194. Drive mechanism 125 may operate to longitudinally translate a plunger through the reservoir, so as to force the liquid drug through an outlet fluid port to needle / cannula 186. Alternatively, other types of drive mechanisms may be used.

In an alternate embodiment, drug delivery device 102 may also include an optional second reservoir 124-2 and second drive mechanism 125-2 which enables the independent delivery of two different liquid drugs. As an example, reservoir 124 may be filled with insulin, while reservoir 124-2 may be filled with glucagon, or Pramlintide, or GLP-1. In some embodiments, each of reservoirs 124, 124-2 may be configured with a separate drive mechanism 125, 125-2, respectively, which may be separately controllable by controller 121 under the direction of MDA 129. Both reservoirs 124, 124-2 may be connected to a common needle / cannula 186.

Drug delivery device 102 may be optionally configured with a user interface 127 providing a means for receiving input from the user and a means for outputting information to the user. User interface 127 may include, for example, light-emitting diodes, buttons on a housing of drug delivery device 102, a sound transducer, a micro-display, a microphone, an accelerometer for detecting motions of the device or user gestures (e.g., tapping on a housing of the device) or any other type of interface device that is configured to allow a user to enter information and/or allow drug delivery device 102 to output information for presentation to the user (e.g., alarm signals or the like).

Drug delivery device 102 includes a patient interface 186 for interfacing with the user to deliver the liquid drug. Patient interface may be, for example, a needle or cannula for delivering the drug into the body of the user (which may be done subcutaneously, intraperitoneally, or intravenously). Drug delivery device 102 may further include a mechanism for inserting the needle / cannula 186 into the body of the user, which may be integral with or attachable to drug delivery device 102. The insertion mechanism may comprise, in one embodiment, an actuator that inserts the needle / cannula 186 under the skin of the user and thereafter retracts the needle, leaving the cannula in place. The actuator may be triggered by user device 105 or may be a manual firing mechanism comprising springs or other energy storing mechanism, that causes the needle / cannula 186 to penetrate the skin of the user.

In one embodiment, drug delivery device 102 includes a communication interface 126, which may be a transceiver that operates according to one or more radio-frequency protocols, such as Bluetooth®, Wi-Fi, near-field communication, cellular, or the like. The controller 121 may, for example, communicate with user device 105 and an analyte sensor 108 via the communication interface 126.

In some embodiments, drug delivery device 102 may be provided with one or more sensors 184. The sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between the patient interface 186 and reservoir 124. The pressure sensor may be coupled to or integral with the actuator for inserting the patient interface 186 into the user. In an example, the controller 121 may be operable to determine a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount. In one embodiment, analyte sensor 108 may be integral with drug delivery device 102.

Drug delivery device 102 further includes a power source 128, such as a battery, a piezoelectric device, an energy harvesting device, or the like, for supplying electrical power to controller 121, memory 123, drive mechanisms 125 and/or other components of drug delivery device 102.

Drug delivery device 102 may be configured to perform and execute processes required to deliver doses of the medication to the user without input from the user device 105 or the optional accessory device 106. As explained in more detail, MDA 129 may be operable, for example, to determine an amount of insulin to be delivered, IOB, insulin remaining, and the like and to cause controller 121 to activate drive mechanism 125 to deliver the medication from reservoir 124. MDA 129 may take as input data received from the analyte sensor 108 or from user app 160.

The reservoirs 124, 124-2 may be configured to store drugs, medications or therapeutic agents suitable for automated delivery, such as insulin, Pramlintide, GLP-1, co-formulations of insulin and GLP-1, glucagon, morphine, blood pressure medicines, arthritis drugs, chemotherapy drugs, fertility drugs, hormonal drugs, or the like.

Drug delivery device 102 may be a wearable device and may be attached to the body of a user, such as a patient or diabetic, at an attachment location and may deliver any therapeutic agent, including any drug or medicine, such as insulin or the like, to a user at or around the attachment location. A surface of drug delivery device 102 may include an adhesive to facilitate attachment to the skin of a user.

When configured to communicate with an external device, such as the user device 105 or the analyte sensor 108, drug delivery device 102 may receive signals over the wired or wireless link 194 from the user device 105 or from the analyte sensor 108. The controller 121 of drug delivery device 102 may receive and process the signals from the respective external devices as well as implementing delivery of a drug to the user according to a diabetes treatment plan or other drug delivery regimen.

*Accessory Device*

Optional accessory device 106 may be, a wearable smart device, for example, a smart watch (e.g., an Apple Watch®), smart eyeglasses, smart jewelry, a global positioning system-enabled wearable, a wearable fitness device, smart clothing, or the like. Accessory device 106 may alternatively be a smart insulin pen that works with drug delivery device 102 in managing blood glucose and treating diabetes of a user. Similar to user device 105, the accessory device 106 may also be configured to perform various functions including controlling or communicating with drug delivery device 102. For example, the accessory device 106 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down version of user app 160 with reduced functionality. In some instances, accessory device 107 may also include sensors of various types.

*Analyte Sensor*

The analyte sensor 108 may include a controller 131, a memory 132, a sensing/measuring device 133, an optional user interface 137, a power source/energy harvesting circuitry 134, and a communication interface 135. The analyte sensor 108 may be communicatively coupled to the processor 151 of the management device 105 or controller 121 of drug delivery device 102. The memory 132 may be configured to store information and programming code 136.

The analyte sensor 108 may be configured to detect one or multiple different analytes, such as glucose, lactate, ketones, uric acid, sodium, potassium, alcohol levels or the like, and output results of the detections, such as measurement values or the like. The analyte sensor 108 may, in an exemplary embodiment, be configured as a continuous glucose monitor (CGM) to measure a blood glucose values at a predetermined time interval, such as every 5 minutes, every 1 minute, or the like. The communication interface 135 of analyte sensor 108 may have circuitry that operates as a transceiver for communicating the measured blood glucose values to the user device 105 over a wireless link 195 or with drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an application-specific integrated circuit, a microcontroller or processor that executes software instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in FIG. 1 as separate from drug delivery device 102, in various embodiments, the analyte sensor 108 and drug delivery device 102 may be incorporated into the same unit. That is, in various examples, the analyte sensor 108 may be a part of and integral with drug delivery device 102 and contained within the same housing as drug delivery device 102 or an attachable housing thereto. In such an example configuration, the controller 121 may be able to implement the functions required for the proper delivery of the medication alone without any external inputs from user device 105, the cloud-based services 111, another sensor (not shown), the optional accessory device 106, or the like.

*Cloud-Based Services*

Drug delivery system 100 may communicate with or receive services from a cloud server 122 providing cloud-based services 111. Services provided by cloud server 112 may include data storage that stores personal or anonymized data, such as blood glucose measurement values, historical IOB or TDI, prior carbohydrate-compensation dosage, and other forms of data. In addition, the cloud-based services 111 may process anonymized data from multiple users to provide generalized information related to TDI, insulin sensitivity, IOB and the like. The communication link 115 that couples the cloud server 112 to other components of system 100, for example, devices 102, 105, 106, 108 of system 100 may be a cellular link, a Wi-Fi link, a Bluetooth® link, or a combination thereof.

*Communication Links*

The wireless communication links 115 and 191-196 may be any type of wireless link operating using known wireless communication standards or proprietary standards. As an example, the wireless communication links 191-196 may provide communication links based on Bluetooth®, Zigbee®, Wi-Fi, a near-field communication standard, a cellular standard, or any other wireless protocol via the respective communication interfaces 126, 135, 154 and 174.

*Operational Example*

In an operational example, user application 160 implements a graphical user interface that is the primary interface with the user and may be used to activate drug delivery device 102, trigger a needle/cannula insertion, start and stop drug delivery device 102, program basal and bolus calculator settings for manual mode as well as program settings specific for automated mode (hybrid closed-loop or closed-loop).

User app 160, provides a graphical user interface 158 that allows for the use of large text, graphics, and on-screen instructions to prompt the user through the set-up processes and the use of system 100. It may also be used to program the user's custom basal insulin delivery profile, accept a recommended basal insulin delivery profile, check the status of drug delivery device 102, initiate bolus doses of insulin, make changes to a patient's insulin delivery profile, handle system alerts and alarms, or allow the user to switch between automated mode and manual mode.

User app 160 may be configured to operate in a manual mode in which user app 160 will deliver insulin at programmed basal rates and user-defined bolus amounts with the option to set temporary basal profiles. The controller 121 will also have the ability to function as a sensor-augmented pump in manual mode, using sensor glucose data provided by the analyte sensor 108 to populate the bolus calculator.

User app 160 may be configured to operate in an automated mode in which user app 160 supports the use of one or multiple target blood glucose values that may be adjusted manually or automatically by the system. For example, in one embodiment, target blood glucose values can range from 110-150 mg/dL, in 10 mg/dL increments, in 5 mg/dL increments, or other increments, but preferably 10 mg/dL increments. The experience for the user will reflect current setup flows whereby the healthcare provider assists the user to program basal rates, glucose targets and bolus calculator settings. These in turn will inform the user app 160 for insulin dosing parameters. The insulin dosing parameters will be adapted over time based on the total daily insulin (TDI) delivered during each use of drug delivery device 102. A temporary hypoglycemia protection mode or an activity mode may be implemented by the user for various time durations in automated mode. With hypoglycemia protection mode or an activity mode, the algorithm reduces insulin delivery and is intended for use over temporary durations when insulin sensitivity is expected to be higher, such as during exercise or fasting.

The user app 160 (or MDA 129) may provide periodic insulin micro-boluses based upon past glucose measurements and/or a predicted glucose over a prediction horizon (e.g., 60 minutes). Optimal post-prandial control may require the user to give meal boluses in the same manner as current pump therapy, but normal operation of the user app 160 will compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

In some embodiments, user device 105 and the analyte sensor 108 may not communicate directly with one another. Instead, data (e.g., blood glucose readings) from analyte sensor may be communicated to drug delivery device 102 via link 196 and then relayed to user device 105 via link 194. In some embodiments, to enable communication between analyte sensor 108 and user device 105, the serial number of the analyte sensor must be entered into user app 160.

User app 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value, and insulin on board (IOB). IOB is estimated by user app 160 taking into account any manual bolus and insulin delivered by the algorithm.

*Description of Embodiments*

The glucose concentration of a typical user of ADD system 100 can be impacted by a wide range of factors. Although an accurate model of the user's glucose concentrations may not be well implemented, the average mismatch between an assumed model and the user's actual glucose concentrations should, over time, be relatively consistent, assuming that the model is not modified and that the physiology or lifestyle of the user does not undergo major changes. Therefore, a significant, sudden increase in the mismatch between the typical deviation from the model predictions and the user's actual glucose levels can provide important insights to a potential issue with the user's insulin delivery, such as a pump occlusion or other site issues. The user's typical deviation from the model predictions can therefore be used to establish a baseline from which further deviations may be indicative of a possible occlusion in the fluid path of ADD system 100.

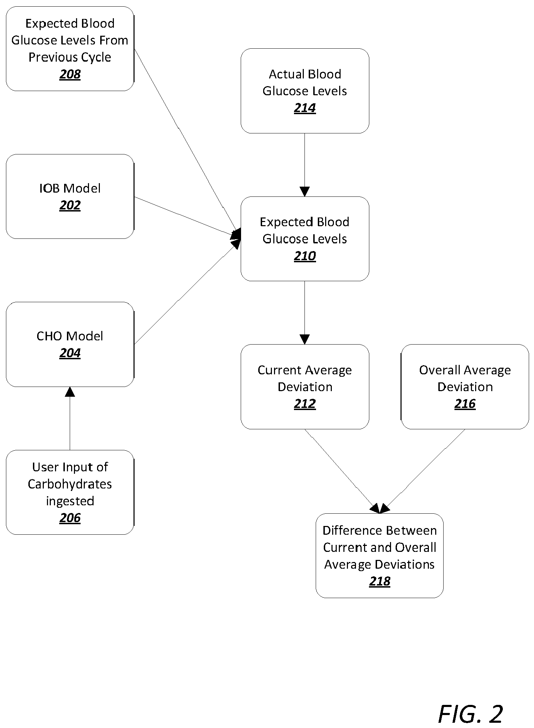

As shown in **FIG. 2,** the disclosed method 200 uses two models, an IOB model 202 to determine the expected changes in the user's blood glucose levels over time based on insulin-on-board, and a CHO (carbohydrate) model 204 to determine the expected changes in the user's blood glucose levels over time based on ingested carbohydrates. The models are evaluated every cycle of MDA 129 which, as previously described, operates on predetermined time interval cycles, in particular 5-minute cycles, during which a new blood glucose reading is received from sensor 108.

IOB model 202 determines the expected impact of the user's insulin delivery history on the user's current glucose concentrations, based on the typical impact for a typical user. The model may be implemented by the following equations:

$$\Delta G_{IOB}(k) = -0.04 \cdot IOB(k-1) \cdot \frac{1800}{TDI(k)}$$

$$(1)$$

where 0.04 is a tunable parameter (in this case, 4%) that represents how much insulin in the body (IOB) is used by, or reduced in, the body in a 5-minute cycle. In this case, a value of 0.04 for the parameter may correspond to a reduction of approximately 50% of the remaining IOB over 90 minutes. The IOB at any cycle may be calculated by the following equation:

$$IOB(k) = \begin{bmatrix} I_{dev,basal}(k-36), I_{dev,basal}(k-35) \dots I_{dev,basal}(k-1) \end{bmatrix} \begin{bmatrix} 1/36 \\ 2/36 \\ 3/36 \\ \vdots \\ 36/36 \end{bmatrix}$$

$$(2)$$

where past insulin delivery deviations from basal over an exemplary past 36 cycles are summed up, and each term is multiplied by a fraction in a matrix operation to account for the fact that the insulin has decayed (in this case, linear decay is assumed). The basal amount against which the deviation is calculated may correspond to the system's assumption of the user's basal needs and may be a value that is entered by the user directly, or a value automatically calculated based on prior insulin delivery history of the user. In the case of the use of prior insulin history, captured as the user's Total daily insulin, the insulin deviation can thus be calculated as follows:

$$I_{dev,basal}(k-1) = I(k-i) - \frac{TDI(k-i)}{48} \cdot \frac{1}{12}$$

(3)

where past insulin delivery deviations from basal are calculated, and "48" is the number of 5-minute cycles in 4 hours. Each equation is explained in further detail below.

As previously noted, MDA 129, as described above as part of ADD system 100 operates using 5-minute cycles. That is, a new blood glucose reading is typically received from sensor 108 at 5-minute intervals, at which time IOB model 202 and CHO model 204 described below are reevaluated. However, the invention is not meant to be limited thereby. Any time interval for reevaluating models 202, 204, whether tied to the receipt of a new blood glucose reading or not, is intended to be within the scope of the invention.

In IOB model 202, Eq. (1) gives the user's drop in blood glucose during each cycle during which the model is evaluated due to the consumption or reduction of insulin-on-board (IOB). Eq. (1) expresses a typical linear decay of insulin-on-board per each cycle. In Eq. (2) and Eq. (3), IOB model 202 calculates the deviation between the user's previous insulin delivery history and the user's expected basal insulin delivery per each cycle of MDA 129, converting this into the user's current estimated insulin-on-board. In each cycle of MDA 129, the model assumes that an estimated 4% of the user's insulin-on-board should be consumed by the body, given the estimated half-time of typical fast-acting insulin as 90 minutes. Specifically, a continuous application of a 4% reduction over 5 minutes will result in a reduction of approximately 50% from the initial value after 90 minutes. The consumed insulin-on-board can then be converted into an estimated glucose drop per consumed insulin via the use of an estimated correction factor, represented by the fraction in Eq. (1), which is a ratio of units of insulin to mg/dL of change of the user's blood glucose level. In the proposed equation, this value is calculated via a commonly used heuristic against the user's total daily insulin, but this term may also be customized for each user or directly entered by the user.

Next, a CHO model 204 estimates the effect on the user's blood glucose levels of the user's previous meal ingestion, where available. The model uses an estimated half-time of typical carbohydrate ingestion of 2.5 hours (150 minutes or 30 5-minute cycles), with similar considerations above (where a repeated reduction of 2.25% per 5 minutes results in a 50% reduction against the initial value after 150 minutes):

$$\Delta G_{CHO}(k) = 0.0225 \cdot M_c(k-1) \cdot 3.5$$

(4)

$$M_c(k) = [CHO(k-60)\ CHO(k-59\ ...\ CHO(k-1)] \begin{bmatrix} 1/60 \\ 2/60 \\ 3/60 \\ \vdots \\ 60/60 \end{bmatrix}$$

(5)

Here, Eq. (4) gives the user's expected rise in blood glucose due to ingestion of a meal. In certain embodiments, the user may provide, via user app 160, an indication 206 of the amount of carbohydrates which were ingested. Based on the typical heuristics of the correction factor and the insulin to carbohydrate (I:C) ratio, the typical increase in glucose concentration per 1g of consumed carbohydrates is approximately 3.5 mg/dL/g of consumed carbohydrates. Further, an estimated 2.25% of remaining carbs on board is estimated to be ingested by the body each cycle based on a half time of 2.5 hours. These variables are exemplary and can be adjusted up or down.

Finally, Eq. (5) provides a method to incorporate the sum of the impact of previous carbohydrate ingestions over the last 5 hours in a single value from which the actual consumption of the ingested meals in the previous cycle can be calculated. This model of remaining ingested carbohydrates is calculated by a multiplication of the total quantity of ingested carbohydrates in the previous 5 hours, or 60 5-minute cycles, and is multiplied by a decaying factor that is reduced for each cycle based on the length of time that has passed since the ingestion, in a vector operation. In this equation, a linear decay over 5 hours is expressed; however, any reasonable shape and duration of the carbohydrate decay curve can be utilized.

The estimates provided by IOB model 202 and CHO model 204 are combined with the value of the expected blood glucose levels from the previous cycle 208 to calculate the expected blood glucose level 210 for the current cycle, in accordance with Eq. (6).

$$G_{exp}(k) = G_{exp}(k-1) + \Delta G_{IOB}(k) + \Delta G_{CHO}(k)$$

$$(6)$$

Finally, in each cycle, the user's current average deviation 212 over a previous predetermined period of time between the expected blood glucose level 210 and the user's actual glucose concentration 214 can be calculated in accordance with Eq. (7). In preferred embodiments, the previous predetermined period of time is 30 minutes (6 cycles of MDA 129); however, any previous period of time could be used.

$$M_{30}(k) = \frac{\sum_{i=1}^{6} G(k-i) - G_{exp}(k-1)}{6}$$

$$(7)$$

where "6" is the number of cycles in a 30-minute period.

This current average deviation 212 can then be compared against the user's overall average deviation 216 in each cycle in accordance with Eq. (8). If the current average deviation 212 is significantly and persistently higher than the user's overall average deviation 216, a potential site issue can be signaled. In one embodiment, if the difference 218 between the current average deviation 212 and the overall average deviation is more than 2 standard deviations from the mean of the overall average deviation 216, an alarm may be raised; however, any similar threshold for raising the alarm can be used.

$$J_{occ}(k)$$
$$= \begin{cases} 1 & M_{30}(k) > \dfrac{\sum_{i=1}^{288} G(k-i) - G_{exp}(k-i)}{288} + 2 \cdot STD\big(G(k-1\dots288) - G_{exp}(k-1\dots288)\big) \\ 0 & otherwise \end{cases}$$

$$(8)$$

where:

   $2 \cdot STD$ is two standard deviations, and
   $J_{occ}$ is a binary flag indicating to the ADD system that a potential site issue has been identified.

Alternately, the probability of whether an issue exists may be provided calculated as in equation (9), with the stated probability linearly increasing from 0 to 100% or higher as the deviation in the previous 30 minutes $M_{30}(k)$ exceeds 2 standard deviations from the typical offsets, as follows:

$$j_{p,occ}(k)$$
$$= \frac{M_{30}(k)}{\dfrac{\sum_{i=1}^{288} G(k-1) - G_{exp}(k-1)}{288} + 2 \cdot STD\big(G(k-1\dots288) - G_{exp}(k-1\dots288)\big)}$$

$$(9)$$

In Eq. (9), an issue may be determined to exist if $J_{p,occ}$ exceeds a certain threshold of probability (such as 75%) or may simply be indicated as an event once it exceeds the full 100% probability.
If it is determined an issue exists, in accordance with Eq. (8) or Eq. (9), ADD system 100 may notify the user by one of several different means. For example, user application 160 may provide an alarm to the user via the user interface 158 of user device 105. The alarm may be, for example, a message displayed on a default screen of the user app 160. The message may be, for example, in the form of a modal window. The alarm may also comprise, in addition to or in lieu of the message display, an audible or haptic signal which may be sensed by the user. In the event that user device 105 is not present in ADD system 100, drug delivery device 102 may deliver the alarm, which may be in the form of a visual alarm, for example, blinking lights on the housing of drug delivery device 102, an audible alarm, for example, a series of beeps issued by drug delivery device 102, or haptic feedback, for example, a series of vibrations created by a vibratory element in the housing of the regular device 102. The alarm may inform the user of the problem and may suggest a course of action, for example, replacing drug delivery device 102.

An occlusion in the fluid delivery path of drug delivery device 102 will typically cause a rise in the user's blood glucose levels because less insulin is being delivered to the user than required. However, other issues with drug delivery device 102 may cause the user's blood glucose levels to fall (or rise), for example, a defect in the drive mechanism 125 which may cause an excess (or insufficient amount) of insulin to be delivered to the user. In this case, two negative standard deviations in Eq. (8), indicating an unusual decline in the user's blood glucose level may indicate an issue with drive mechanism 125 as opposed to an occlusion in the patient interface 186. Such issues may also be flagged to the user.

The IOB model 202 given by Eqs. (1-3) and the CHO model 204 given by Eqs. (4-5) are based on average of a large population of users. The actual applicability of the models to the user in question is not as important as the user's deviations from these models, given by Eqs. (6-7), which should be consistent on a day-to-day basis. A deviation from the user's typical deviation from the models may be indicative of a problem.

Note that negative deviations (e.g., the impact of unknown exercise) are automatically incorporated as this detection algorithm and the pump occlusion both only consider increases in glucose concentrations.

The equations describing the models above assume that this method is implemented by ADD system 100, which uses 5-minute cycles. For example, the constant "288" in Eq. (8) denotes a 24-hour period. Use with other ADD systems or with ADD system 100 described herein using different durations of cycles is contemplated be within the scope of the invention.

It should be noted that the IOB model 202 and the CHO model 204 described herein by Eqs. (1-3) and Eqs. (4-5) respectively are exemplary in nature. It is contemplated that the invention could use any reasonable models to estimate the effect of insulin-on-board and carbohydrates ingested with respect to the use of blood glucose levels and to measure the user's deviation from those models. An alarm is only raised when the current deviation differs greatly from the user's typical or average deviation from the models.

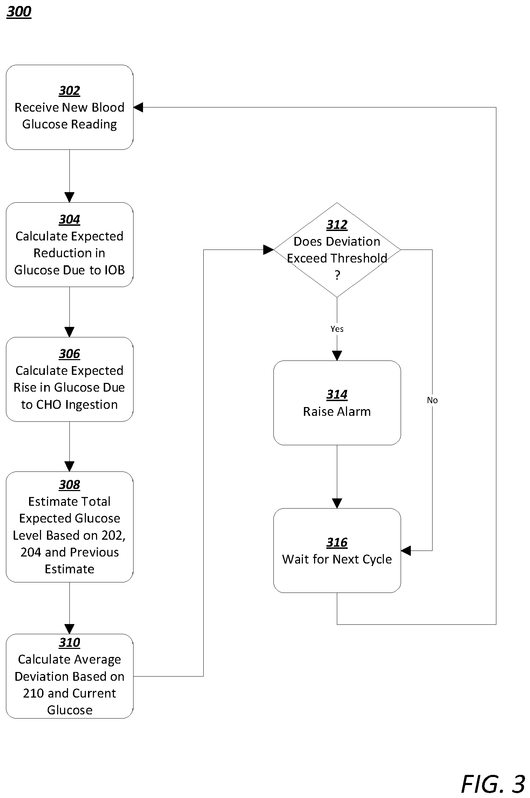

FIG. 3 is a flowchart showing the process 300 described above. The process is repeated for each cycle in MDA 129 which, in the exemplary embodiments described with respect to ADD system 100 herein, occur in 5-minute intervals. At step 302, a new blood glucose reading is received from sensor 108. At this time, the IOB model 202 is reevaluated. At step 304, the IOB model 202 described by Eqs. (1-3) is executed to calculate the expected reduction in the user's blood glucose levels due to the remaining insulin-on-board, that is, the insulin remaining in the body of the user. At step 306, the CHO model 204 described by Eqs. (4-5) is executed to calculate the expected change in the user's blood glucose values based on previous meal ingestion of the user. At step 308, the total expected value 210 of the user's blood glucose level for the current cycle is estimated in accordance with Eq. (6). The user's expected blood glucose value for the current cycle 210 is calculated as a sum of the user's expected blood glucose level from the previous cycle 208, the change in the user's blood glucose levels due to insulin-on-board, as calculated by the IOB model 202 and the change in the user's blood glucose levels due to ingested carbohydrates, as calculated by CHO model 204. At step 310, the user's actual current average deviation 212 from the expected glucose values for the past 30 minutes is calculated in accordance with Eq. (7). As would be realized, actual deviations calculated for other time periods are contemplated to be within the scope of the invention. At decision point 312, is determined if the difference between the current average deviation 212 and the overall average deviation 216 exceeds a predetermined deviation threshold, which, in the embodiment described herein is two standard deviations over a 24-hour period, from the values predicted by the models and the user's current blood glucose reading from sensor 108, in accordance with Eq. (8). If the difference 218 between the current average deviation 212 and the overall average deviation 216 exceeds the deviation threshold (in either direction) then an alarm is raised at step 314. The methods of raising the alarm have been previously described. As would be realized, the two standard deviation threshold is exemplary only. Any other deviation threshold may be used to determine whether the alarm should be raised. If, at decision point 312, the difference 218 does not exceed the deviation threshold, no alarm is raised and control proceeds to step 216 to wait for the next cycle of MDA 129.

The described process 300 may be part of MDA 129 which may execute on drug delivery device 102, on user device 105 or partially on drug delivery device 102 and partially on user device 105. The alarms raised to the user may be delivered via user interface 158 on user device 105, by output device 155 on user device 105, on drug delivery device 102, or by a combination of any of the foregoing. The alarm may further be raised by user interface 178 on accessory device 106 which may be, for example, a smartwatch or other smart accessory device.

As would be realized by one of skill in the art, many variations on the embodiments disclosed herein are possible. In particular, various models and intervals and thresholds used in the models are contemplated to be the within the scope of the invention and the invention is not meant to be limited by the specific embodiments disclosed herein.

Although the present invention has been described above and is defined in the claims, it should be understood that the present invention can also be defined according to the following embodiments:

1. A method comprising:

periodically determining an expected blood glucose level of user of an automatic drug delivery system based on one or more models,

determining an average deviation from the expected blood glucose reading given by the models over a predetermined period of time,

determining, based on a current blood glucose level of the user, an actual deviation from the expected blood glucose levels given by the models, and

raising an alarm to the user if the actual deviation exceeds the average deviation by a predetermined threshold.

2. The method according to embodiment 1, wherein the one or more models includes an IOB model for determining the effect on the expected blood glucose level of the user due to insulin-on-board.

3. The method according to embodiment 1 or 2, wherein the IOB model calculates the deviation between an insulin delivery history of the user, and a periodic expected basal insulin delivery and converts the deviation into a current estimate of insulin-on-board.

4. The method according to one of embodiments 1 to 3, wherein the IOB model assumes that a predetermined percentage of the insulin-on-board is consumed during each periodic cycle during which the IOB model is evaluated.

5. The method according to one of embodiments 1 to 4, wherein the consumed insulin-on-board is converted into an estimated drop in the blood glucose level of the user per consumed unit of insulin via the use of an estimation correction factor.

6. The method according to one of embodiments 1 to 5, wherein the one or more models includes a CHO model for determining the effect on the expected blood glucose level of the user due to previous ingestion of carbohydrates by the user.

7. The method according to embodiment 6, wherein the CHO model uses an estimate of the increase in glucose concentration per gram of consumed carbohydrates.

8. The method according to embodiment 6, wherein the CHO model uses an estimate of a percentage of consumed carbohydrates used during each cycle during which the CHO model is evaluated.

9. The method according to one of embodiments 6 to 8, wherein the expected blood glucose level is calculated periodically during each cycle of a medication delivery algorithm of the automatic drug delivery system and further wherein the expected blood glucose level is based on an IOB model for determining an expected change in the blood glucose level due to insulin-on-board and a CHO model for determining an expected change in the blood glucose level due to consumed carbohydrates.

10. The method according to one of embodiments 1 to 9, wherein the expected blood glucose level is further based on the calculated expected blood glucose level from a previous cycle of the medication delivery algorithm.

11. The method according to one of embodiments 1 to 10, wherein the actual deviation from the expected blood glucose level is calculated over a predetermined period of time.

12. The method according to one of embodiments 1 to 11, wherein the alarm is raised if the actual deviation calculated over the predetermined period of time exceeds an average deviation calculated over a previous 24-hour time period by a predetermined threshold.

13. The method according to one of embodiments 1 to 12, wherein the predetermined threshold is two standard deviations.

14. The method according to one of embodiments 1 to 13, wherein the alarm is raised via a user interface of the user device portion of the automatic drug delivery system.

15. The method according to one of embodiments 1 to 14, wherein the alarm comprises a message displayed on a user interface of the user device portion, an audible alarm generated by the user device portion, a haptic signal generated by the user device portion, or combination of any of the foregoing.

16. The method according to one of embodiments 1 to 15, wherein the alarm comprises a visual, audible or haptic signal generated by a drug delivery device portion of the automatic drug delivery system.

17. An automatic drug delivery system comprising:

a processor,

software, implementing a medication delivery algorithm executing on the processor, software performing the functions of

periodically determining an expected blood glucose level of the user of the automatic drug delivery system based on one or more models,

determining an average deviation from the expected blood glucose reading given by the models over a predetermined period of time,

determining, based on a current blood glucose level of the user, an actual deviation from the expected blood glucose levels given by the models, and

raising an alarm to the user if the actual deviation exceeds the average deviation by a predetermined threshold.

18. The system according to embodiment 17, wherein the expected blood glucose level is calculated periodically during each cycle of the medication delivery algorithm and further wherein the expected blood glucose level is based on an IOB model for determining the expected change in the blood glucose level due to insulin-on-board and a CHO model for determining the expected change in the blood glucose level due to consumed carbohydrates.

19. The system according to embodiment 17 or 18, wherein the expected blood glucose level is further based on the calculated expected blood glucose level from a previous cycle of the medication delivery algorithm.

20. The system according to one of embodiments 17 to 19, wherein the actual deviation from the expected blood glucose level is calculated over a predetermined period of time, and wherein the alarm is raised if the actual deviation calculated over the predetermined period of time exceeds the average deviation calculated over a previous 24-hour period by a predetermined threshold.

21. The system according to one of embodiments 17 to 20, wherein the alarm comprises a visual, audible or haptic signal generated by the automatic drug delivery system.

22. An automatic drug delivery system comprising:

at least one processor;

software, implementing a medication delivery algorithm executing on the at least one processor, the software performing the functions of:

periodically determining an expected blood glucose level (210) of a user of the automatic drug delivery system based on one or more models;

determining an average deviation (216) from the expected blood glucose level (210) given by the models over a predetermined period of time;

determining, based on an actual or current blood glucose level (214) of the user, an actual or current deviation (212) from the expected blood glucose level (210) given by the models; and

raising an alarm to the user if the actual deviation exceeds the average deviation by a predetermined threshold.

23. The system of embodiment 22, wherein the one or more models includes an insulin-on-board (IOB) model (202) for determining the effect on the expected blood glucose level (210) of the user due to insulin-on-board (IOB).

24. The system of embodiment 23, wherein the insulin-on-board (IOB) model (202) calculates a deviation between an insulin delivery history of the user and a periodic expected basal insulin delivery, and converts the deviation into a current estimate of insulin-on-board.

25. The system of embodiment 22 or 23 wherein the insulin-on-board (IOB) model (202) assumes that a predetermined percentage of the insulin-on-board is consumed during each periodic cycle during which the insulin-on-board (IOB) model (202) is evaluated.

26. The system of embodiment 25, wherein the consumed insulin-on-board is converted into an estimated drop in the blood glucose level of the user per consumed unit of insulin via the use of an estimated correction factor.

27. The system of one of embodiments 22 to 26, wherein the one or more models includes a carbohydrate (CHO) model (204) for determining the effect on the expected blood glucose level of the user due to previous ingestion of carbohydrates by the user.

28. The system of embodiment 27, wherein the carbohydrate (CHO) model (204) uses an estimate of the increase in glucose concentration per gram of consumed carbohydrates.

29. The system of embodiment 27 or 28, wherein the carbohydrate (CHO) model (204) uses an estimate of a percentage of consumed carbohydrates used during each cycle during which the carbohydrate (CHO) model (204) is evaluated.

30. The system of one of embodiments 22 to 29, wherein the expected blood glucose level is calculated periodically during each cycle of a medication delivery algorithm of the automatic drug delivery system and further wherein the expected blood glucose level is based on an insulin-on-board (IOB) model (202) for determining an expected change in the blood glucose level due to insulin-on-board and a carbohydrate (CHO) model (204) for determining an expected change in the blood glucose level due to consumed carbohydrates.

31. The system of one of embodiments 22 to 30, wherein the expected blood glucose level is based or further based on the calculated expected blood glucose level from a previous cycle of the medication delivery algorithm.

32. The system of one of embodiments 22 to 31, wherein the actual deviation from the expected blood glucose level is calculated over a predetermined period of time.

33. The system of one of embodiments 22 to 32, wherein the alarm is raised if the actual deviation calculated over the predetermined period of time exceeds an average deviation calculated over a previous 24-hour period by a predetermined threshold.

34. The system of one of embodiments 22 to 33, wherein the predetermined threshold is a factor multiplied by the standard deviations, wherein the factor is greater than 1.3, in particular wherein the factor is 2.

35. The system of one of embodiments 22 to 34, wherein the alarm is raised via a user interface of a user device portion of the automatic drug delivery system.

36. The system of one of embodiments 22 to 35, wherein the alarm comprises a message displayed on a user interface of the user device portion, an audible alarm generated by the user device portion, a haptic signal generated by the user device portion, or a combination of any of the foregoing.

37. The system of one of embodiments 22 to 36, wherein the alarm comprises a visual, audible or haptic signal generated by a drug delivery device portion of the automatic drug delivery system.

38. The system of one of embodiments 22 to 37, wherein the insulin-on-board (IOB) model (202) and/or the carbohydrate (CHO) model (204) are based on the average of a large population of users, in particular on the average of a population of at least 100 or at least 1000 users.

39. The system of one of embodiments 22 to 38, wherein the system comprises a drug delivery device, a user device and an analyte sensor, wherein the software implementing the medication delivery algorithm is performed by a processor of the drug delivery device, a processor of the user device, or by a processor of the user device in combination with a processor of the drug delivery device.

40. The system of one of embodiments 22 to 39, wherein the system comprises an analyte sensor for measuring the actual or current blood glucose level (214) of the user.

41. The system of one of embodiments 22 to 40, wherein the user's expected blood glucose value (210) for a current cycle is calculated as a sum of the user's expected blood glucose value (208) from the previous cycle, the change in the user's blood glucose levels due to insulin-on-board, as calculated by the insulin-on-board (IOB) model (202) and the change in the user's blood glucose levels due to ingested carbohydrates as calculated by the carbohydrate (CHO) model (204).

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

To those skilled in the art to which the invention relates, many modifications and adaptations of the invention may be realized. Implementations provided herein, including sizes, shapes, ratings, compositions and specifications of various components or arrangements of components, and descriptions of specific manufacturing processes, should be considered exemplary only and are not meant to limit the invention in any way. As one of skill in the art would realize, many variations on implementations discussed herein which fall within the scope of the invention are possible. Moreover, it is to be understood that the features of the various embodiments described herein were not mutually exclusive and can exist in various combinations and permutations, even if such combinations or permutations were not made express herein, without departing from the spirit and scope of the invention. Accordingly, the method and apparatus disclosed herein are not to be taken as limitations on the invention but as an illustration thereof. The scope of the invention is defined by the claims which follow.

**Claims**

1. An automatic drug delivery system, comprising:

   at least one processor;
   software, implementing a medication delivery algorithm executing on the at least one processor, the software performing the functions of:

for multiple consecutive cycles, periodically receiving (302) a user's blood glucose reading from a sensor (108),

periodically, for each cycle, executing (304) an insulin-on-board (IOB) model (202) for calculating the expected reduction in the user's blood glucose level due to remaining insulin-on-board (IOB) of the user, periodically, for each cycle, executing (306) a carbohydrate (CHO) model (204) for calculating the expected change in the user's blood glucose value based on previous meal/carbohydrate ingestion by the user, periodically, for each cycle, estimating (308) the total expected value (210) of the user's blood glucose level by combining the expected reduction in the user's blood glucose level due to remaining insulin-on-board (IOB) of the user obtained by the insulin-on-board (IOB) model (202) and the expected change in the user's blood glucose value based on previous meal/carbohydrate ingestion by the user obtained by the carbohydrate (CHO) model (204),

calculating (310) the user's current average deviation (212) from the expected total glucose value (210) with respect to a first time period which covers multiple consecutive cycles,

calculating (310) the user's overall average deviation (216) from the expected total glucose value (210) with respect to a second time period which covers multiple consecutive cycles, wherein the second time period is longer than the first time period, and

determining (312) if the difference between the current average deviation (212) and the overall average deviation (216) exceeds a predetermined threshold.

2. The system of claim 1, wherein the software performs the functions of raising an alarm (314) if the difference between the current average deviation (212) and the overall average deviation (216) exceeds a predetermined threshold.

3. The system of claim 1 or 2, wherein the software performs the function of waiting for the next cycle, and repeating the functions mentioned in claim 1.

4. The system of claim 1, wherein the cycle covers a time period ranging from 1 to 10 minutes, in particular a time period of 5 minutes,

wherein the first time period covers a time period ranging from 15 minutes to 2 hours, in particular a time period of 30 min, and

wherein the second time period covers a time period ranging from 6 hours to 48 hours, in particular a time period of 24 hours.

5. The system of one of the preceding claims, wherein the insulin-on-board (IOB) model (202) calculates a deviation between an insulin delivery history of the user and a periodic expected basal insulin delivery, and converts the deviation into a current estimate of insulin-on-board.

6. The system of one of the preceding claims, wherein the insulin-on-board (IOB) model (202) assumes that a predetermined percentage of the insulin-on-board is consumed during each periodic cycle during which the insulin-on-board (IOB) model (202) is evaluated.

7. The system of claim 6, wherein the consumed insulin-on-board is converted into an estimated drop in the blood glucose level of the user per consumed unit of insulin via the use of an estimated correction factor.

8. The system of one of the preceding claims, wherein the carbohydrate (CHO) model (204) uses an estimate of the increase in glucose concentration per gram of consumed carbohydrates.

9. The system of one of the preceding claims, wherein the carbohydrate (CHO) model (204) uses an estimate of a percentage of consumed carbohydrates used during each cycle during which the carbohydrate (CHO) model (204) is evaluated.

10. The system of one of the preceding claims, wherein the expected blood glucose level is based or further based on the calculated expected blood glucose level from a previous cycle of the medication delivery algorithm.

11. The system of one of the preceding claims, wherein the alarm is raised if the actual deviation calculated over the predetermined period of time exceeds an average deviation calculated over a previous 24-hour period by a predetermined threshold.

12. The system of one of the preceding claims, wherein the predetermined threshold is a factor multiplied by the standard deviations, wherein the factor is greater than 1.3, in particular wherein the factor is 2.

13. The system of one of the preceding claims, wherein the alarm is raised via a user interface of a user device portion of the automatic drug delivery system, and/or
the alarm comprises a message displayed on a user interface of the user device portion, an audible alarm generated by the user device portion, a haptic signal generated by the user device portion, or a combination of any of the foregoing, and/or wherein the alarm comprises a visual, audible or haptic signal generated by a drug delivery device portion of the automatic drug delivery system.

14. The system of one of the preceding claims, wherein the insulin-on-board (IOB) model (202) and/or the carbohydrate (CHO) model (204) are based on the average of a large population of users, in particular on the average of a population of at least 100 or at least 1000 users.

15. The system of one of the preceding claims, wherein the system comprises a drug delivery device, a user device and an analyte sensor, wherein the software implementing the medication delivery algorithm is performed by a processor of the drug delivery device, a processor of the user device, or by a processor of the user device in combination with a processor of the drug delivery device.

FIG. 1

Expected Blood
Glucose Levels From
Previous Cycle
**208**

Actual Blood
Glucose Levels
**214**

IOB Model
**202**

Expected Blood
Glucose Levels
**210**

CHO Model
**204**

Current Average
Deviation
**212**

Overall Average
Deviation
**216**

User Input of
Carbohydrates
ingested
**206**

Difference Between
Current and Overall
Average Deviations
**218**

*FIG. 2*

*300*

```
    ┌─────────────────┐
    │      302        │
    │ Receive New Blood│◄──────────────────┐
    │ Glucose Reading │                    │
    └─────────────────┘                    │
            │                              │
            ▼                              │
    ┌─────────────────┐                    │
    │      304        │        ┌───────────────────┐
    │ Calculate Expected│      │       312         │
    │ Reduction in    │        │  Does Deviation   │
    │ Glucose Due to IOB│      │ Exceed Threshold  │
    └─────────────────┘        │        ?          │
            │                  └───────────────────┘
            ▼                    │ Yes          │ No
    ┌─────────────────┐          ▼              │
    │      306        │    ┌─────────────┐      │
    │ Calculate Expected│  │     314     │      │
    │ Rise in Glucose Due│ │ Raise Alarm │      │
    │ to CHO Ingestion│    └─────────────┘      │
    └─────────────────┘          │              │
            │                    ▼              │
            ▼              ┌─────────────┐       │
    ┌─────────────────┐    │     316     │◄──────┘
    │      308        │    │ Wait for Next Cycle│
    │ Estimate Total  │    └─────────────┘
    │ Expected Glucose│
    │ Level Based on 202,│
    │ 204 and Previous│
    │   Estimate      │
    └─────────────────┘
            │
            ▼
    ┌─────────────────┐
    │      310        │
    │ Calculate Average│
    │ Deviation Based on│
    │ 210 and Current │
    │   Glucose       │
    └─────────────────┘
```

*FIG. 3*

European Patent Office

**EUROPEAN SEARCH REPORT**

Application Number

EP 23 19 4655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2021 522895 A (INSULET CORP) 2 September 2021 (2021-09-02) * paragraphs [0004], [0034], [0038], [0062], [0075], [0043], [0050] * | 1-15 | INV. G16H20/17 |
| Y | US 2011/054439 A1 (YODFAT OFER [IL] ET AL) 3 March 2011 (2011-03-03) * paragraphs [0006], [0059] * | 1-15 | |
| Y | US 2021/398639 A1 (ROY ANIRBAN [US] ET AL) 23 December 2021 (2021-12-23) * paragraph [0004] * | 14 | |
| A | US 2018/174675 A1 (ROY ANIRBAN [US] ET AL) 21 June 2018 (2018-06-21) * paragraphs [0004], [0032], [0090], [0079], [0100] * | 1-15 | |
| A | CN 111 246 898 B (MEDTRONIC MINIMED INC) 8 April 2022 (2022-04-08) * the whole document * | 1-15 | |
| A | EP 2 174 128 B1 (ROCHE DIAGNOSTICS GMBH [DE]; HOFFMANN LA ROCHE [CH]) 18 May 2016 (2016-05-18) * the whole document * | 1-15 | TECHNICAL FIELDS SEARCHED (IPC) G16H |

The present search report has been drawn up for all claims

| Place of search | Date of completion of the search | Examiner |
|---|---|---|
| Munich | 30 January 2024 | Laub, Christoph |

EPO FORM 1503 03.82 (P04C01)

## ANNEX TO THE EUROPEAN SEARCH REPORT
## ON EUROPEAN PATENT APPLICATION NO.

EP 23 19 4655

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2024

| Patent document cited in search report | | Publication date | Patent family member(s) | | Publication date |
|---|---|---|---|---|---|
| JP 2021522895 | A | 02-09-2021 | AU | 2019263490 A1 | 26-11-2020 |
| | | | AU | 2023210562 A1 | 24-08-2023 |
| | | | CA | 3099113 A1 | 07-11-2019 |
| | | | CN | 112236826 A | 15-01-2021 |
| | | | EP | 3788628 A1 | 10-03-2021 |
| | | | JP | 7124120 B2 | 23-08-2022 |
| | | | JP | 2021522895 A | 02-09-2021 |
| | | | JP | 2022167924 A | 04-11-2022 |
| | | | US | 2019336683 A1 | 07-11-2019 |
| | | | US | 2019336684 A1 | 07-11-2019 |
| | | | US | 2023181824 A1 | 15-06-2023 |
| | | | WO | 2019213493 A1 | 07-11-2019 |
| US 2011054439 | A1 | 03-03-2011 | US | 2011054439 A1 | 03-03-2011 |
| | | | WO | 2009095908 A2 | 06-08-2009 |
| US 2021398639 | A1 | 23-12-2021 | CN | 115917662 A | 04-04-2023 |
| | | | EP | 4169037 A1 | 26-04-2023 |
| | | | US | 2021393876 A1 | 23-12-2021 |
| | | | US | 2021398639 A1 | 23-12-2021 |
| | | | WO | 2021257361 A1 | 23-12-2021 |
| US 2018174675 | A1 | 21-06-2018 | CA | 3046100 A1 | 28-06-2018 |
| | | | CN | 110191731 A | 30-08-2019 |
| | | | EP | 3558422 A1 | 30-10-2019 |
| | | | US | 2018169332 A1 | 21-06-2018 |
| | | | US | 2018169333 A1 | 21-06-2018 |
| | | | US | 2018169334 A1 | 21-06-2018 |
| | | | US | 2018174675 A1 | 21-06-2018 |
| | | | US | 2021043297 A1 | 11-02-2021 |
| | | | US | 2021043298 A1 | 11-02-2021 |
| | | | US | 2021043299 A1 | 11-02-2021 |
| | | | US | 2021043300 A1 | 11-02-2021 |
| | | | US | 2022044784 A1 | 10-02-2022 |
| | | | US | 2023282328 A1 | 07-09-2023 |
| | | | WO | 2018119150 A1 | 28-06-2018 |
| CN 111246898 | B | 08-04-2022 | AU | 2019349568 A1 | 11-03-2021 |
| | | | CA | 3110070 A1 | 02-04-2020 |
| | | | CN | 111246898 A | 05-06-2020 |
| | | | EP | 3856281 A1 | 04-08-2021 |
| | | | JP | 2022502159 A | 11-01-2022 |
| | | | KR | 20210072758 A | 17-06-2021 |
| | | | US | 2020101222 A1 | 02-04-2020 |
| | | | US | 2021093783 A1 | 01-04-2021 |
| | | | US | 2023248910 A1 | 10-08-2023 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 23 19 4655

This annex lists the patent family members relating to the patent documents cited in  the above-mentioned European search report.
The members are as contained in the European Patent Office EDP file on
The European Patent Office is in no way liable for these particulars which are merely given for the purpose of information.

30-01-2024

| Patent document cited in search report | Publication date | Patent family member(s) | | Publication date |
|---|---|---|---|---|
| | | WO 2020068188 A1 | | 02-04-2020 |
| EP 2174128        B1 | 18-05-2016 | DK | 2174128 T3 | 06-06-2016 |
| | | EP | 2174128 A2 | 14-04-2010 |
| | | ES | 2576639 T3 | 08-07-2016 |
| | | US | 2009018406 A1 | 15-01-2009 |
| | | WO | 2009013637 A2 | 29-01-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7303549 B
- US 7137964 B
- US 6740059 B
- US 63359010